(19) Europäisches Patentamt
European Patent Office
Office européen des brevets

(11) Veröffentlichungsnummer: **0 132 729**
**A2**

# (12) EUROPÄISCHE PATENTANMELDUNG

(21) Anmeldenummer: **84108288.6**

(22) Anmeldetag: **14.07.84**

(51) Int. Cl.⁴: **C 07 C 125/065**
A 01 N 47/12

(30) Priorität: **26.07.83 DE 3326873**

(43) Veröffentlichungstag der Anmeldung:
**13.02.85 Patentblatt 85/7**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schwamborn, Michael, Dr.**
**von-Lohe-Strasse 9**
**D-5000 Koeln 80(DE)**

(72) Erfinder: **Hagemann, Hermann, Dr.**
**Kandinsky-Strasse 52**
**D-5090 Leverkusen 1(DE)**

(72) Erfinder: **Heywang, Gerhard, Dr.**
**Nittumer Weg 4**
**D-5060 Bergisch Gladbach 2(DE)**

(72) Erfinder: **Behrenz, Wolfgang, Dr.**
**Untergruendemich 14**
**D-5063 Overath(DE)**

## (54) Halogenalkylcarbamidsäureester, Verfahren zu ihrer Herstellung und ihre Verwendung als Schädlingsbekämpfungsmittel.

(57) Die vorliegende Erfindung betrifft Halogenalkylcarbamidsäureester der allgemeinen Formel (I)

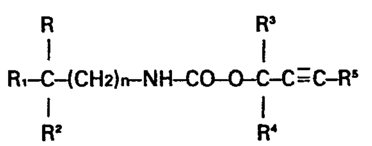

(I)

in welcher

R, $R^1$ und $R^2$ unabhängig voneinander für gegebenenfalls durch Halogen substituiertes Methyl stehen, wobei wenigstens eine der Methylgruppen R, $R^1$ und $R^2$ durch wenigstens ein Halogen-atom substituiert ist, und

$R^3$, $R^4$ und $R^5$ gleich oder verschieden sind und einzeln für Wasserstoff oder für gegebenenfalls substituiertes Alkyl stehen, und

n für 0 oder 1 steht,

welche als Synergisten in Schädlingsbekämpfungsmitteln verwendet werden, die zusätzlich Arthropodizide enthalten, welche vorzugsweise gegen Insekten und Spinnentiere, insbesondere gegen Insekten wirksam sind.

EP 0 132 729 A2

Croydon Printing Company Ltd

0132729

BAYER AKTIENGESELLSCHAFT 5090 Leverkusen, Bayerwerk
Konzernverwaltung RP
Patentabteilung S/by-c
I/III

Halogenalkylcarbamidsäureester, Verfahren zu ihrer Herstellung und ihre Verwendung als Schädlingsbekämpfungsmittel

Die vorliegende Erfindung betrifft Halogenalkylcarbamidsäureester, Verfahren zu ihrer Herstellung sowie ihre Verwendung als Schädlingsbekämpfungsmittel, vorzugsweise zur Bekämpfung von Arthropoden, insbesondere von Insekten, Milben und Spinnentieren.

Synergistische Mischungen von insektiziden Wirkstoffen, z.B. von Pyrethroiden mit bestimmten Methylendioxyphenyl-Derivaten, z.B. Piperonylbutoxid als Synergisten sind bereits bekannt geworden (vgl. z.B. K. Naumann, Chemie der Pflanzenschutz- und Schädlingsbekämpfungsmittel, Springer Verlag Berlin, Band 7 (1981) Seiten 3-6). Ferner sind bestimmte N-Arylcarbamidsäurealkinylester (vgl. DE-A 2 041 986) und bestimmte N-Alkylcarbamidsäurealkinylester (vgl. BE-A- 633 594) als Synergisten beschrieben worden. Die Wirksamkeit dieser Verbindungen ist jedoch unter den in der Praxis auftretenden Bedingungen nicht immer voll befriedigend.

Es wurden nun die neuen Carbamidsäureester der allgemeinen Formel (I)

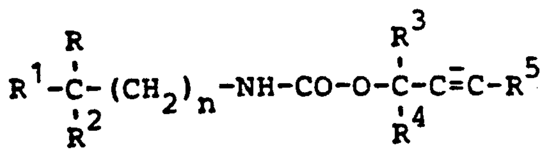

(I)

in welcher

$R, R^1$ und $R^2$ unabhängig voneinander für gegebenenfalls durch Halogen substituiertes Methyl stehen, wobei wenigstens eine der Methylgruppen $R$, $R^1$ und $R^2$ durch wenigstens ein Halogenatom substituiert ist, und

$R^3, R^4$ und $R^5$ gleich oder verschieden sind und einzeln für Wasserstoff oder für gegebenenfalls substituiertes Alkyl stehen, und

n für 0 oder 1 steht,

gefunden.

Die neuen Verbindungen der Formel (I) können als Synergisten in Schädlingsbekämpfungsmitteln verwendet werden, die zusätzlich Arthropodizide enthalten, welche vorzugsweise gegen Insekten und Spinnentiere, insbesondere gegen Insekten wirksam sind.

Als Arthropodizide (gegen Arthropoden wirksame Stoffe) kommen praktisch alle üblichen Wirkstoffe in Frage (vgl. z.B. K.H. Büchel, Pflanzenschutz- und Schädlingsbekämpfungsmittel, Thieme Verlag Stuttgart, 1977, und Farm Chemicals Handbook 1979, Meister Publishing Co, Willougby, 1979).

Weiterhin wurde gefunden, daß man die neuen Carbamidsäureester der allgemeinen Formel (I)

$$R^1-\underset{R^2}{\overset{R}{C}}-(CH_2)_n-NH-CO-O-\underset{R^4}{\overset{R^3}{C}}-C\equiv C-R^5 \qquad (I)$$

in welcher

$R$, $R^1$ und $R^2$ unabhängig voneinander für gegebenenfalls durch Halogen substituiertes Methyl stehen, wobei wenigstens eine der Methylgruppen $R$, $R^1$ und $R^2$ durch wenigstens ein Halogenatom substituiert ist, und

$R^3$, $R^4$ und $R^5$ gleich oder verschieden sind und einzeln für Wasserstoff oder für gegebenenfalls substituiertes Alkyl stehen, und

n für 0 oder 1 steht

erhält, wenn man Alkylisocyanate der allgemeinen Formel (II)

$$R^1-\overset{\displaystyle R}{\underset{\displaystyle R^2}{C}}-(CH_2)_n-NCO \qquad (II)$$

in welcher

$R, R^1, R^2$ und n die oben angegebene Bedeutung haben

mit Alkinylalkoholen der allgemeinen Formel (III)

$$HO-\overset{\displaystyle R^3}{\underset{\displaystyle R^4}{C}}-C\equiv C-R^5 \qquad (III)$$

in welcher

$R^3, R^4$ und $R^5$ die oben angegebene Bedeutung haben,

gegebenenfalls in Gegenwart eines aprotischen Verdünnungsmittels und gegebenenfalls in Gegenwart eines Katalysators bei Temperaturen zwischen 0 und 120°C umsetzt.

Die gegebenenfalls durch Halogen substituierten Methylgruppen R, $R^1$ und $R^2$ sind gleich oder verschieden und enthalten 1 bis 3, vorzugsweise 1 oder 2 und ganz besonders bevorzugt 1 Halogenatom je Methylgruppe, wobei die Halogenatome gleich oder verschieden sein können. Als Halogenatome stehen Fluor, Chlor, Brom und/oder Iod, vorzugsweise Fluor und/oder Chlor.

Bevorzugt stehen R, $R^1$ und $R^2$ für $CH_3$, $CH_2Cl$ und/oder $CH_2F$, wobei wenigstens eine der Gruppen R, $R^1$ und $R^2$ $CH_2Cl$ oder $CH_2F$ bedeutet. Besonders bevorzugt sind die Halogenatome in den Methylgruppen R, $R^1$ und $R^2$ gleich.

Als ganz besonders bevorzugte Molekülteile $RR^1R^2C-$ seien aufgeführt: $(CH_3)_2CH_2FC-$, $CH_3(CH_2F)_2C-$, $(CH_2F)_3C-$, $(CH_3)_2CH_2ClC-$, $CH_3(CH_2Cl)_2C-$ und $(CH_2Cl)_3C-$.

Wenn n für 0 steht, ist im Molekül der Formel (I) die $RR^1R^2C$-Gruppe direkt an die NH-Gruppe gebunden. Wenn n für 1 steht, ist die $RR^1R^2C$-Gruppe über eine $CH_2$-Gruppe mit der NH-Gruppe verbunden.

Als gegebenenfalls substituiertes Alkyl $R^3$, $R^4$ und $R^5$ steht geradkettiges oder verzweigtes gegebenenfalls substituiertes Alkyl mit vorzugsweise 1-8, insbesondere 1-5 und besonders bevorzugt 1-3 Kohlenstoffatomen. Beispielhaft seien gegebenenfalls substituiertes Methyl, Ethyl, n- und i-Propyl sowie n-, iso- und tert.-Butyl genannt, wobei diese Reste bevorzugt unsubstituiert sind.

Die in der Definition von $R^3$ und $R^4$ genannten Alkylreste können einen oder mehrere, vorzugsweise 1 bis 3, insbesondere 1 oder 2 gleiche oder verschiedene Substituenten tragen. Als Substituenten seien beispielsweise aufgeführt:

Alkoxy mit vorzugsweise 1 bis 4, insbesondere 1 oder 2 Kohlenstoffatomen, wie Methoxy, Ethoxy, n- und iso-Propyloxy und n-, iso- und tert.-Butyloxy; Alkylthio mit vorzugsweise 1 bis 4, insbesondere 1 oder 2 Kohlenstoffatomen, wie Methylthio, Ethylthio, n- und iso-Propylthio und n-, iso- und tert.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Halogen, vorzugsweise Fluor, Chlor, Brom und Jod, insbesondere Chlor und Brom; Cyano; Nitro; Alkoxycarbonyl mit vorzugsweise 2 bis 4, insbesondere 2 oder 3 Kohlenstoffatomen, wie Methoxycarbonyl und Ethoxycarbonyl.

Der in der Definition von $R^5$ genannte Alkylrest kann einen oder mehrere, vorzugsweise 1 bis 3, insbesondere 1 oder 2 gleiche oder verschiedene Substituenten tragen. Als Substituenten seien beispielhaft aufgeführt:

Alkoxy mit vorzugsweise 1 bis 4, insbesondere 1 oder 2 Kohlenstoffatomen, wie Methoxy, Ethoxy, n- und iso-Propyloxy und n-, iso- und tert.-Butyloxy; Alkylthio mit vorzugsweise 1 bis 4, insbesondere 1 oder 2 Kohlenstoffatomen, wie Methylthio, Ethylthio, n- und iso-Propylthio und n-, iso- und tert.-Butylthio; Halogenalkyl mit vorzugsweise 1 bis 4, insbesondere 1 oder 2

Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Halogen, vorzugsweise Fluor, Chlor, Brom und Jod, insbesondere Chlor und Brom; Cyano; Nitro; Alkoxycarbonyl mit vorzugsweise 2 bis 4, insbesondere 2 oder 3 Kohlenstoffatomen, wie Methoxycarbonyl und Ethoxycarbonyl.

Vorzugsweise sind die Reste $R^3$, $R^4$ und $R^5$ unsubstituiert. Besonders bevorzugt stehen $R^3$, $R^4$ und $R^5$ für Wasserstoff.

Bevorzugt enthalten die Methylgruppen R, $R^1$ und $R^2$, welche durch Halogen substituiert sind ein Halogenatom als Substituenten.

Bevorzugt werden die Carbamidsäureester der allgemeinen Formel (I), in welcher

R,$R^1$ und $R^2$ unabhängig voneinander für gegebenenfalls durch Fluor oder Chlor substituiertes Methyl stehen, wobei wenigstens eine der Methylgruppen R, $R^1$ und $R^2$ durch wenigstens ein Fluor- oder Chloratom substituiert ist, und

$R^3$,$R^4$,$R^5$ gleich oder verschieden sind und einzeln für Wasserstoff oder für einen gegebenenfalls durch Halogen, Cyano, $C_1$-$C_4$-Alkoxy, $C_1$-$C_4$-

Alkylthio oder Trifluormethyl substituierter Alkylrest mit 1-2 Kohlenstoffatomen stehen, und

n für 0 oder 1 steht.

Die Erfindung betrifft besonders bevorzugt Carbamidsäureester der allgemeinen Formel (I), in welcher

$R$, $R^1$ und $R^2$ unabhängig voneinander für $CH_3$, $CH_2Cl$ und/oder $CH_2F$ stehen, wobei wenigstens einer der Reste $R$, $R^1$ und $R^2$ für $CH_2Cl$ oder $CH_2F$ steht, und

$R^3$, $R^4$ und $R^5$ für Wasserstoff stehen, und

n für 0 oder 1 steht.

Verwendet man beispielsweise Bis-2,2-fluormethyl-3-fluorpropylisocyanat und Propargylalkohol als Ausgangsstoffe, so kann das erfindungsgemäße Verfahren durch folgendes Formelschema erläutert werden:

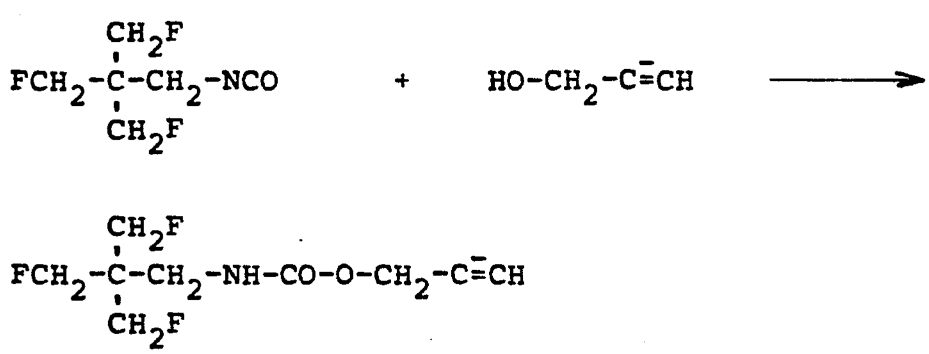

Beim erfindungsgemäßen Verfahren können die Ausgangsmaterialien vorzugsweise in äquimolaren Mengen eingesetzt werden. Es ist jedoch auch möglich eine der Komponenten gleichzeitig als Verdünnungsmittel zu verwenden.

Beim erfindungsgemäßen Verfahren sind praktisch alle aprotischen Lösungsmittel als Verdünnungsmittel geeignet. Bevorzugt seien hier Ketone, wie Aceton, gegebenenfalls halogenierte aliphatische oder aromatische Kohlenwasserstoffe, wie Methylenchlorid oder Toluol, Nitrile, wie Acetonitril sowie deren Gemische genannt. Das Verfahren kann jedoch auch ohne die Verwendung eines Lösungsmittels durchgeführt werden.

Als Katalysatoren werden dem Reaktionsgemisch gegebenenfalls Triethylamin, Diazabicyclooctan (DABCO) oder Dibutylzinnlaurat zugesetzt.

Die Reaktionstemperatur wird beim erfindungsgemäßen Verfahren zwischen etwa 0 und 120°C, vorzugsweise zwischen etwa 0 und 60°C gehalten. Das Verfahren wird vorzugsweise bei Normaldruck durchgeführt.

Die Ausgangsverbindungen der Formel (II) sind bekannt und/oder können in üblicher Weise nach an sich bekannten Verfahren und Methoden hergestellt werden (vgl. z.B. Methoden der organischen Chemie (Houben-Weyl-Müller) 4. Auflage, Band 8 (1952) S. 119 ff. DE-A- 20 45 906 und DE-A- 20 45 907).

Die neuen Isocyanate der Formel (II), in welchen R, $R^1$ und/oder $R^2$ für fluorierte Methylgruppen und n für 0 stehen, werden aus den entsprechenden Chlorpivaloylchloriden erhalten. Hierzu setzt man die bekannten Pivaloylchloride ($RR^1R^2C-COCl$), in welchen R, $R^1$ und/oder $R^2$ chlorierte Methylgruppen bedeuten, mit Alkalimetallfluoriden, wie Kaliumfluorid, bei erhöhter Temperatur, z.B. bei 200°C in einem organischen Lösungsmittel, wie Tetramethylensulfon um, wobei bezogen auf die Zahl der durch Fluor zu ersetzenden Chloratome (Chloratome der Methylgruppen und Chloratom des Säurechloridteiles), die jeweils entsprechende äquimolare Menge Alkalifluorid, oder ein geringer Überschuß, eingesetzt wird. Die auf diese Weise erhaltenen Fluorpivaloylfluoride werden in bekannter Weise (vgl. z.B. Methoden der Organischen Chemie, Houben-Weyl, Band IX/1 (1957), Seiten 867-872, Thieme-Verlag, Stuttgart) in die Isocyanate übergeführt. Hierzu werden die Fluorpivaloylfluoride z.B. in einer Lösung in Aceton mit etwa molaren Mengen Natriumazid (in wäßriger Lösung) bei etwa 0 bis 20°C umgesetzt. Anschließend wird mit Toluol extrahiert, die Toluolphase abgetrennt, mit Natriumsulfat getrocknet und die Toluol-Lösung zum Sieden erhitzt. Die entstandenen Isocyanate der Formel (II) können direkt in der Toluol-Lösung umgesetzt werden oder durch Destillation isoliert werden.

Die neuen Isocyanate der Formel (II), in welchen R, $R^1$ und/oder $R^2$ für fluorierte Methylgruppen und n für 1 stehen, können ebenfalls aus den oben erwähnten

Fluorpivaloylfluoriden (oder entsprechenden Fluorpivaloylchloriden) erhalten werden.

Hierzu werden die Fluorpivaloylsäurehalogenide ($RR^1R^2C$ CO Hal, Hal ist vorzugsweise F) mit wäßriger Ammoniaklösung bei etwa 0 bis 10°C in Methylenchlorid behandelt, wobei die Fluorpivalsäureamide ($RR^1R^2C-CONH_2$) ausfallen. Die Pivalsäureamide werden abgesaugt und getrocknet und anschließend mit Phosphorpentoxid bei Temperaturen von etwa 160 bis 200°C unter vermindertem Druck (ca. 70 mbar) umgesetzt, wobei die Fluorpivalsäurenitrile ($RR^1R^2C-C\equiv N$) entstehen, welche durch Abdestillieren isoliert werden. Diese Nitrile werden anschließend mit Wasserstoff in Gegenwart eines Katalysators (wie Raney-Nickel) bei ca. 80 bis 90 bar und Temperaturen von etwa 35 bis 50°C in Methanol zu den Neopentylaminen ($RR^1R^2-CH_2-NH_2$) hydriert, welche durch Abfiltrieren des Katalysators und nachfolgendem Abdestillieren des Lösungsmittels erhalten werden. Diese Neopentylamine (gegebenenfalls in Form der Hydrochloride) werden durch die Umsetzung mit Phosgen ($COCl_2$) z.B. in Chlorbenzol bei Temperaturen zwischen 20°C und dem Siedepunkt des Reaktionsgemisches zu den Neopentylisocyanaten umgesetzt. Die Neopentylisocyanate ($RR^1R^2C-CH_2-NCO$) werden durch Destillation isoliert und gegebenenfalls gereinigt. Die Umsetzungen der Fluorpivaloylsäurehalogenide ($RR^1R^2CCOHal$) zu den Neopentylisocyanaten ($RR^1R^2C-CH_2-NCO$) können auch vom vorstehenden abweichend in den einzelnen Stufen unter

Anwendung allgemein üblicher anderer Methoden der organischen Chemie vorgenommen werden.

Die Ausgangsverbindungen der Formel (III) sind bekannt und/oder können in üblicher Weise nach an sich bekannten Verfahren hergestellt werden (vgl. W. Reppe J. Liebigs Ann. Chem. 569, 1 (1955), W. Ried, Angew. Chem. 76, 973 (1964), W. Ried et al. Chem. Ber. 98, 245 (1965)).

Die Carbamidsäureester der allgemeinen Formel (I) weisen in Mischung mit Stoffen beliebiger Konstitution, welche gegen Arthropoden wirksam sind, starke synergistische Wirkungen auf, welche ihre Verwendung in Schädlingsbekämpfungsmitteln ermöglicht.

Bevorzugt werden die Carbamidsäureester der allgemeinen Formel (I) zusammen mit arthropodiziden

1. Carbaminsäureestern und/oder

2. Carbonsäureestern einschließlich der natürlichen sowie synthetischen Pyrethroide und/oder

3. Phosphorverbindungen, wie Phosphorsäure- und Phosphonsäureestern, einschließlich der Thio- und Dithioverbindungen und/oder

4. Halogen-(cyclo)-alkanen, wie z.B. Hexachlorcyclohexan

verwendet.

Überraschenderweise ist die Wirkung der neuen erfindungsgemäßen Wirkstoffkombinationen gegen Arthropoden wesentlich höher als die Wirkung der Einzelkomponenten bzw. die Summe der Wirkungen der Einzelkomponenten. Sie ist ferner wesentlich höher als die Wirkung von Wirkstoffkombinationen mit dem bekannten handelsüblichen Synergisten Piperonylbutoxid. Die erfindungsgemäß verwendbaren Carbamidsäureester zeigen außerdem ausgezeichnete synergistische Wirksamkeit nicht nur bei einer Wirkstoffklasse, sondern bei Wirkstoffen aus den verschiedensten chemischen Stoffgruppen.

Die synergistische Wirkung der Verbindungen der allgemeinen Formel (I) zeigt sich besonders bevorzugt bei

1) Carbamidsäureestern der Formel (IV)

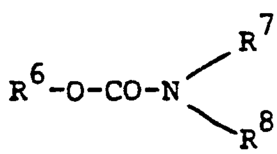

(IV)

in welcher

$R^6$ für einen gegebenenfalls substituierten carbocyclischen oder heterocyclischen aromatischen Rest oder für einen gegebenenfalls substituierten Oximrest steht (wobei die weiter unten erläuterten Reste $R^6$ bevorzugt werden),

$R^7$ für $C_1$-$C_4$-Alkyl steht und

$R^8$ für Wasserstoff, $C_1$-$C_4$-Alkyl oder für einen Rest Y steht, wobei

Y für den Rest -CO-$R^9$ steht, worin

$R^9$ für Halogen, $C_1$-$C_4$-Alkyl, $C_1$-$C_4$-Alkoxy, $C_3$-$C_5$-Alkenoxy, $C_3$-$C_5$-Alkinoxy, $C_1$-$C_4$-Alkylthio, $C_1$-$C_4$-Alkyl-amino, Di-$C_1$-$C_4$-Alkylamino, $C_1$-$C_4$-Alkyl-hydroxylamino,

für gegebenenfalls durch Halogen, Nitro, Cyano, Trifluormethyl, $C_1$-$C_4$-Alkyl, $C_1$-$C_4$-Alkoxy, $C_1$-$C_4$-Alkylendioxy, $C_1$-$C_4$-Alkylthio, $C_1$-$C_4$-Alkoxy-carbonyl substituiertes Phenoxy, Phenylthio oder Phenylamino, für 2,3-Dihydro-2,2-dimethyl-7-benzofuranyl oder für den Rest

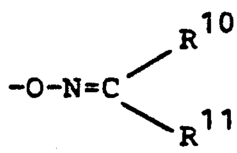

steht, worin

$R^{10}$ für Wasserstoff, $C_1$-$C_4$-Alkyl oder Di-$C_1$-$C_4$-alkyl-amino-carbonyl steht und

$R^{11}$ für $C_1$-$C_4$-Alkyl, $C_1$-$C_4$-Alkylthio, Cyano-$C_1$-$C_4$-alkylthio, $C_1$-$C_4$-Alkylthio-$C_1$-$C_4$-alkyl steht,

oder die beiden Reste $R^{10}$ und $R^{11}$ zusammen für gegebenenfalls durch Sauerstoff, Schwefel, SO oder $SO_2$ unterbrochenes $C_2$-$C_8$-Alkandiyl stehen, oder

in welcher

Y für den Rest $-S_n(O)_m-R^{12}$ steht, worin

n für 1 oder 2 und

m für 0, 1 oder 2 stehen und

$R^{12}$ für gegebenenfalls durch Halogen substituiertes $C_1$-$C_4$-Alkyl, $C_3$-$C_5$-Alkenyl, $C_3$-$C_5$-Alkinyl oder $C_3$-$C_6$-Cycloalkyl, für gegebenenfalls durch Halogen, Cyano, Nitro, Trifluormethyl, Trifluormethoxy, Trifluormethylthio, $C_1$-$C_4$-Alkyl oder $C_1$-$C_4$-Alkoxy substituiertes Phenyl, Benzyl oder Phenylethyl oder für den Rest

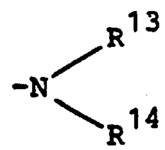

steht, worin

$R^{13}$ für $C_1$-$C_4$-Alkyl, $C_3$-$C_5$-Alkenyl, $C_3$-$C_5$-Alkinyl, $C_3$-$C_6$-Cycloalkyl oder Benzyl steht und

$R^{14}$ für $C_1$-$C_4$-Alkyl, $C_3$-$C_5$-Alkenyl, $C_3$-$C_5$-Alkinyl, $C_3$-$C_6$-Cycloalkyl, Benzyl, Phenylethyl, Halogen-carbonyl, Formyl, $C_1$-$C_4$-Alkyl-carbonyl, $C_1$-$C_4$-Alkoxy-carbonyl, $C_1$-$C_4$-Alkoxyphenoxy-carbonyl, $C_3$-$C_5$-Alkinoxy-carbonyl, $C_3$-$C_5$-Alkenoxycarbonyl, $C_1$-$C_4$-Alkylthiocarbonyl, $C_1$-$C_4$-Alkyl-amino-carbonyl, $C_1$-$C_4$-Alkyl-hydroxylamino-carbonyl, $C_1$-$C_{10}$-Alkyl-phenoxycarbonyl, Di-$C_1$-$C_4$-alkyl-amino-carbonyl, Phenylthiocarbonyl, Phenoxycarbonyl, 2,3-Dihydro-2,2-dimethyl-7-benzofuranyloxycarbonyl, für gegebenenfalls durch Halogen, Cyano, Nitro, Trifluormethyl, $C_1$-$C_{10}$-Alkyl oder $C_1$-$C_4$-Alkoxy substituiertes Phenylsulfenyl, Phenylsulfinyl, Phenylsulfonyl oder Phenyl steht, oder für den Rest

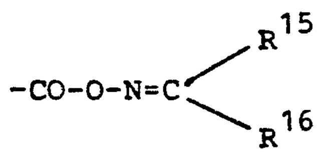

steht, worin

$R^{15}$ die oben für $R^{10}$ angegebene Bedeutung und

$R^{16}$ die oben für $R^{11}$ angegebene Bedeutung hat,

wobei ferner im Rest 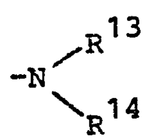 die Reste $R^{13}$ und $R^{14}$ zusammen für eine gegebenenfalls durch Sauerstoff oder

Schwefel unterbrochene Kohlenwasserstoffkette mit 3 bis 8 Kohlenstoffatomen stehen und worin weiter $R^{12}$ auch für den gleichen Rest stehen kann, an den der Rest $-S_n(O)_m-R^{12}$ gebunden ist.

Als Wirkstoffkomponenten ganz besonders bevorzugt sind Carbamidsäureester der Formel (IV), in welcher

$R^6$ für gegebenenfalls durch $C_1$-$C_4$-Alkyl, $C_2$-$C_4$-Alkenyl, $C_1$-$C_4$-Alkoxy, $C_1$-$C_4$-Alkoxy-methyl, $C_1$-$C_4$-Alkylthio, $C_1$-$C_4$-Alkylthio-methyl, $C_1$-$C_4$-Alkylamino, Di-($C_1$-$C_4$-alkyl)-amino, Di-($C_3$-$C_4$-alkenyl)-amino, Halogen, Dioxolanyl, Methylendioxy und/oder durch den Rest $-N{=}CH{-}N(CH_3)_2$ substituierte Reste aus der Reihe Phenyl, Naphthyl, 2,3-Dihydro-7-benzofuranyl, Pyrazolyl oder Pyrimidinyl steht, oder in welcher

$R^6$ für einen Alkylidenaminorest der Formel (IVa)

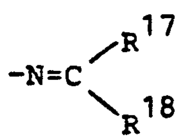

(IVa)

steht, in welcher

$R^{17}$ und $R^{18}$ die oben für $R^{10}$ bzw. $R^{11}$ angegebene Bedeutung haben, und

$R^7$ für $C_{1-4}$-Alkyl steht und

$R^8$ für Wasserstoff oder $C_1$-$C_4$-Alkyl (vorzugsweise für Wasserstoff) steht.

Als Beispiele für die Carbamidsäureester der Formel (IV) seien genannt: 2-Methyl-phenyl-, 2-Ethyl-phenyl-, 2-iso-Propyl-phenyl-, 2-sec-Butyl-phenyl-, 2-Methoxy-phenyl-, 2-Ethoxy-phenyl-, 2-iso-Propoxy-phenyl-, 4-Methyl-phenyl-, 4-Ethyl-phenyl-, 4-n-Propyl-phenyl-, 4-Methoxy-phenyl-, 4-Ethoxy-phenyl-, 4-n-Propoxy-phenyl-, 3,4,5-Trimethyl-phenyl-, 3,5-Dimethyl-4-methylthio-phenyl-, 3-Methyl-4-dimethylaminophenyl-, 2-Ethylthiomethyl-phenyl-, 1-Naphthyl-, 2,3-Dihydro-2,2-dimethyl-7-benzofuranyl, 2,3-(Dimethyl-methylendioxy)-phenyl-, 2-(4,5-Dimethyl-1,3-dioxolan-2-yl)-phenyl-, 1-Methylthio-ethyliden-amino-, 2-Methylthio-2-methylpropylidenamino-, 1-(2-Cyano-ethylthio)-ethylidenamino- und 1-Methylthiomethyl-2,2-dimethyl-propylidenamino-N-methyl-carbamidsäureester.

Die synergistische Wirkung der Verbindungen der allgemeinen Formel (I) zeigt sich weiter bevorzugt bei

2) Carbonsäureestern der Formel (V)

$$R^{19}\text{-CO-O-}\underset{}{\overset{R^{20}}{\overset{|}{C}}}\text{H-}R^{21} \qquad \text{(V)}$$

in welcher

$R^{19}$ für einen offenkettigen oder cyclischen Alkylrest steht, der gegebenenfalls substituiert ist durch Halogen, Alkyl, Cycloalkyl, durch gegebenenfalls durch Halogen, Alkyl und/oder Alkoxy substituiertes Alkenyl, durch Phenyl oder Styryl, welche gegebenenfalls durch Halogen, gegebenenfalls Halogen-substituierte Reste aus der Reihe Alkyl, Alkoxy, Alkylendioxy und/oder Alkylthio substituiert sind, durch spirocyclisch verknüpftes, gegebenenfalls Halogen-substituiertes Cycloalk(en)yl, welches gegebenenfalls benzannelliert ist, in welcher weiter

$R^{20}$ für Wasserstoff, Alkyl, Halogenalkyl, Alkenyl, Alkinyl oder Cyano steht, und

$R^{21}$ für einen gegebenenfalls substituierten Alkyl- oder Arylrest oder für einen Heterocyclus steht, oder zusammen mit $R^{20}$ und dem Kohlenstoffatom, an das beide Reste gebunden sind, einen Cyclopentenonring bildet.

Ganz besonders als Wirkstoffkomponenten bevorzugt sind Carbonsäureester der Formel (V), in welcher

$R^{19}$ für den Rest

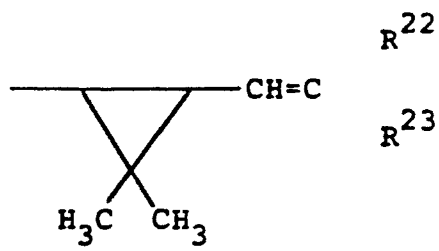

steht, worin

$R^{22}$ für Wasserstoff, Methyl, Fluor, Chlor oder Brom und

$R^{23}$ für Methyl, Fluor, Chlor, Brom, $C_1$-$C_2$-Fluoralkyl oder $C_1$-$C_2$-Chlorfluoralkyl oder für gegebenenfalls durch Halogen und/oder gegebenenfalls Halogen-substituierte Reste der Reihe $C_1$-$C_4$-Alkyl, $C_1$-$C_4$-Alkoxy, $C_1$-$C_4$-Alkylthio und/oder $C_1$-$C_2$-Alkylendioxy substituiertes Phenyl steht, oder worin beide Reste $R^{22}$ und $R^{23}$ für $C_2$-$C_5$-Alkandiyl (Alkylen) stehen; oder in welcher

$R^{19}$ für den Rest $-\underset{R^{25}}{\underset{|}{CH}}-R^{24}$ steht, worin

$R^{24}$ für gegebenenfalls durch Halogen und/oder durch gegebenenfalls Halogen-substituierte Reste der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und

$R^{25}$ für Isopropyl oder Cyclopropyl steht;

oder in welcher

$R^{19}$ für einen der Reste

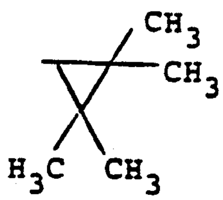 , 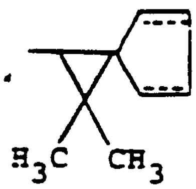 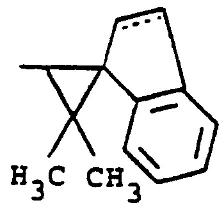

wobei die gepunkteten Linien mögliche Doppelbindungen andeuten sollen, oder für Methyl steht, und in welcher weiter

$R^{20}$ für Wasserstoff, $C_1$-$C_4$-Alkyl, $C_1$-$C_4$-Halogenalkyl, Cyano oder Ethinyl steht und

$R^{21}$ für die Reste der Reihe Phenyl, Furyl oder Tetrahydrophthalimido steht, wobei diese Reste substituiert sein können durch Halogen und/oder Reste der Reihe $C_1$-$C_4$-Alkyl, $C_2$-$C_4$-Alkenyl, $C_1$-$C_4$-Alkoxy, $C_2$-$C_4$-Alkenoxy, $C_1$-$C_4$-Alkylthio, $C_1$-$C_2$-Alkylendioxy, Phenoxy und/oder Benzyl, welche ihrerseits durch Halogen substituiert sein können und wobei $R^{21}$ vorzugsweise für Pentafluorphenyl, 3,4-Dichlorphenyl, Phenoxyphenyl, welches in einem oder beiden Phenylringen durch Halogen substituiert sein kann oder für Tetrahydrophthalimido steht.

Weiter sind die natürlich vorkommenden Pyrethroide (wie Pyrethreum) als Carbonsäureester der Formel (V) besonders bevorzugt.

Als Beispiele für die Carbonsäureester der Formel (V) seien genannt:

Essigsäure-(2,2,2-trichlor-1-(3,4-dichlor-phenyl)-ethyl)-ester, 2,2-Dimethyl-3-(2-methyl-propen-1-yl)-cyclopropan-carbonsäure-(3,4,5,6-tetrahydro-phthalimido-methyl)-ester, 2,2-Dimethyl-3-(2,2-dichlor-vinyl)-cyclopropan-carbonsäure-(3-phenoxy-benzyl)-ester, 2,2-Dimethyl-3-(2,2-dichlorvinyl)-cyclopropan-carbonsäure-(α-cyano-3-phenoxy-benzyl)-ester, 2,2-Dimethyl-3-(2,2-dichlorvinyl)-cyclopropancarbonsäure-(α-cyano-4-fluor-3-phenoxy-benzyl)-ester, 2,2-Dimethyl-3-(2,2-dichlorvinyl)-cyclopropancarbonsäure-(pentafluor-benzyl)-ester, 2,2-Dimethyl-3-(2,2-dibromvinyl)-cyclopropancarbonsäure-(α-cyano-3-phenoxy-benzyl)-ester und 3-Methyl-2-(4-chlor-phenyl)-butansäure-(α-cyano-3-phenoxy-benzyl)-ester.

Weiter zeigt sich die synergistische Wirkung der Verbindungen der allgemeinen Formel (I) bevorzugt bei

3) Phosphorsäure- und Phosphonsäureestern der allgemeinen Formel (VI)

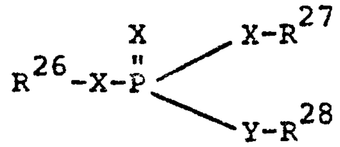

(VI)

in welcher

X jeweils für O oder S steht und

Y für O, S, -NH- oder für eine direkte Bindung zwischen dem zentralen P-Atom und $R^{28}$ steht und

$R^{26}$ und $R^{27}$ gleich oder verschieden sind und für gegebenenfalls substituiertes Alkyl oder Aryl stehen,

$R^{28}$ für Wasserstoff, gegebenenfalls substituiertes Alkyl, Aryl, Heteroaryl, Aralkyl, Alkenyl, Dioxanyl oder einen Oximrest oder für den gleichen Rest steht, an den es gebunden ist.

Besonders bevorzugt sind Phosphorsäure- und Phosphonsäureester der Formel (VI), in welcher

$R^{26}$ und $R^{27}$ gleich oder verschieden sind und für $C_1$-$C_4$-Alkyl oder Phenyl stehen,

$R^{28}$ für Wasserstoff, Alkyl mit 1 bis 4 Kohlenstoffatomen steht, das gegebenenfalls durch Halogen, Hydroxyl, Cyano, gegebenenfalls Halogen-substituiertes Phenyl, Carbamoyl, Alkylsulfonyl, Alkylsulfinyl, Alkylcarbonyl, Alkoxy, Alkylmercapto,

Alkoxycarbonyl, Alkylaminocarbonyl, letztere mit jeweils bis zu 6 Kohlenstoffatomen, substituiert ist, für Alkenyl mit bis zu 4 Kohlenstoffatomen, das gegebenenfalls durch Halogen, gegebenenfalls Halogen-substituiertes Phenyl oder $C_1$-$C_4$-Alkoxycarbonyl substituiert ist, oder für den Rest der allgemeinen Formel (VIa)

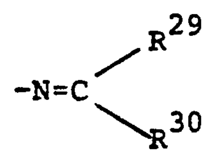

(VIa)

steht,

wobei $R^{29}$ und $R^{30}$ die oben für $R^{10}$ bzw. $R^{11}$ angegebene Bedeutung besitzen, oder für Cyano oder Phenyl stehen, und in welcher

$R^{28}$ ferner für Dioxanyl, das durch denselben Rest substituiert ist, an den $R^{27}$ gebunden ist, oder $R^{28}$ für den gleichen Rest, an den es gebunden ist, oder $R^{28}$ für Phenyl, das gegebenenfalls durch Methyl, Nitro, Cyano, Halogen und/oder Methylthio substituiert ist steht und $R^{28}$ außerdem besonders bevorzugt für gegebenenfalls durch $C_1$-$C_4$-Alkoxy, $C_1$-$C_4$-Alkylthiomethyl, $C_1$-$C_4$-Alkyl und/oder durch Halogen substituierte heteroaromatische Reste, wie Pyridinyl, Chinolinyl, Chinoxalinyl, Pyrimidinyl oder Benzo-1,2,4-triazinyl steht.

Im einzelnen seien genannt:

O,O-Dimethyl- bzw. O,O-Diethyl-O-(2,2-dichlor- bzw. 2,2-dibromvinyl)-phosphorsäureester,
O,O-Diethyl-O-(4-nitro-phenyl)-thionophosphorsäureester
O,O-Dimethyl-O-(3-methyl-4-methylthio-phenyl)-thionophosphorsäureester
O,O-Dimethyl-O-(3-methyl-4-nitro-phenyl)-thionophosphorsäureester,
O-Ethyl-S-n-propyl-O-(2,4-dichlorphenyl)-thionophosphorsäureester,
O-Ethyl-S-n-propyl-O-(4-methylthio-phenyl)-thionophosphorsäureester,
O,O-Dimethyl-S-(4-oxo-1,2,3-benzothriazin(3)yl-methyl)-thionothiolphosphorsäureester,
O-Methyl-O-(2-iso-propyl-6-methoxy-pyrimidin(4)yl)-thionomethanphosphonsäureester,
O,O-Diethyl-O-(2-iso-propyl-6-methyl-pyrimidin(4)yl)-thionophosphorsäureester,
O,O-Diethyl-O-(3-chlor-4-methyl-cumarin(7)yl)-thionophosphorsäureester,
O,O-Dimethyl-2,2,2-trichlor-1-hydroxy-ethan-phosphonsäureester,
O,O-Dimethyl-S-(methylaminocarbonyl-methyl)-thionophosphorsäureester.

Weiterhin zeigt sich die synergistische Wirkung der Verbindungen der allgemeinen Formel (I) bevorzugt bei

4) Halogen(cyclo)-alkanen, wie z.B. Hexachlorcyclohexan, 1,1,1-Trichlor-2,2-bis-(4-chlorphenyl)-ethan, 1,1,1-Trichlor-2,2-bis-(4-methoxyphenyl)-ethan und 1,1-Dichlor-2,2-bis-(4-ethylphenyl)-ethan.

Die Gewichtsverhältnisse der Synergisten und Wirkstoffe können in einem relativ großen Bereich variiert werden. Im allgemeinen werden die als Synergisten verwendeten Verbindungen der Formel (I) mit den übrigen Wirkstoffen in Mischungsverhältnissen zwischen 1 : 100 und 100 : 1, vorzugsweise zwischen 1 : 5 und 5 : 1 (Gewichtsteile) eingesetzt.

Die erfindungsgemäßen Wirkstoffkombinationen besitzen nicht nur eine schnelle knock-down-Wirkung, sondern bewirken auch die Abtötung der tierischen Schädlinge, insbesondere von Insekten und Milben, die in der Landwirtschaft, in Forsten, im Vorrats- und Materialschutz sowie im Hygienebereich vorkommen. Sie sind gegen normal sensible und resistente Arten sowie gegen alle oder einzelne Entwicklungsstadien wirksam.

Zu den tierischen Schädlingen, welche unter Verwendung der Verbindungen der Formel (I) bekämpft werden können, gehören beispielsweise:

Aus der Ordnung der Isopoda z.B. Oniscus asellus, Porcellio scaber.
Aus der Ordnung der Thysanura z.B. Lepisma saccharina.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Schistocerca gregaria.
Aus der Ordnung der Dermaptera z.B. Forficula auricularia.
Aus der Ordnung der Isoptera z.B. Reticulitermes spp.
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Heteroptera z.B. Cimex lectularius, Rhodnius prolixus, Triatoma spp.
Aus der Ordnung der Homoptera z.B. Myzus spp., und Psylla spp.
Aus der Ordnung der Lepidoptera z.B. Ephestia kuehniella und Galleria mellonella.
Aus der Ordnung der Coleoptera z.B. Anobium punctatum, Rhizopertha dominica, Bruchidius obtectus, Acanthoscelides obtectus, Hylotrupes bajulus, Oryzaephilus surinamensis, Sitophilus spp., Dermestes spp., Trogoderma spp., Anthrenus spp., Attagenus spp., Lyctus spp., Ptinus spp., Niptus hololeucus, Gibbium psylloides, Tribolium spp. und Tenebrio molitor.
Aus der Ordnung der Hymenoptera z.B. Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp.,

Chrysomyia spp., Gastrophilus spp., Hyppobosca spp., Stomoxys spp., Oestrus spp., Hypoderma spp. und Tabanus spp.
Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Boophilus spp., Rhipicephalus spp., Amblyomma spp., Hyalomma spp., Ixodes spp., Psoroptes spp., Chorioptes spp., Sarcoptes spp.

Die Wirkstoffkombinationen aus den Verbindungen der Formel (I) und den übrigen Wirkstoffen können in die üblichen Formulierungen übergeführt werden, wie Lösungen, Emulsionen, Spritzpulver, Suspensionen, Pulver, Stäubemittel, Schäume, Pasten, lösliche Pulver, Aerosole, Suspensions-Emulsionskonzentrate, Wirkstoffimprägnierte Natur- und synthetische Stoffe, Feinstverkapselungen in polymeren Stoffen, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffgemische mit Streckmitteln, also flüssigen Lösungsmitteln, unter Druck stehenden verflüssigten Gasen und/oder festen

Trägerstoffen, gegebenenfalls unter Verwendung von oberflächenaktiven Mitteln, also Emulgiermitteln und/ oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte

natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel: nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate; als Dispergiermittel: z.B. Lignin- und Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe, und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoffkombination, vorzugsweise zwischen 0,5 und 90 %.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der gesamte Wirkstoffgehalt (einschließlich Synergist) der aus den handelsüblichen Formulierungen bereiteten Anwendungsformen kann in weiten Bereichen variieren. Die Wirkstoffkonzentration der Anwendungsformen kann von 0,001 bis zu 95 Gew.-% Wirkstoffkombination, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Anhand der folgenden biologischen Beispiele soll die Wirksamkeit der erfindungsgemäß verwendbaren Verbindungen der Formel (I) erläutert werden:

Angewendete Testmethode:

$LT_{100}$-Test

Testtiere: Musca domestica ♀♀, Stamm Weymanns (resistent)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapier von 9,5 cm Durchmesser pipettiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Tiere wird bis zu 6 Stunden fortlaufend kontrolliert. Es wird diejenige Zeit ermittelt, die für eine 100 %ige knock down-Wirkung erforderlich ist. Wird die $LT_{100}$ nach 6 Stunden nicht erreicht, wird der % Satz der knock down gegangenen Testtiere festgestellt.

Wirkstoffe, Synergisten, die Konzentrationen der Wirkstoffe, Synergisten und Gemische sowie ihre Wirkungen gehen aus den nachfolgenden Tabellen hervor.

Tabelle 1

Beispiele für erfindungsgemäß verwendbare Wirkstoffe

A. 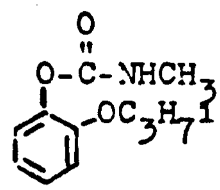 (Propoxur)

B. 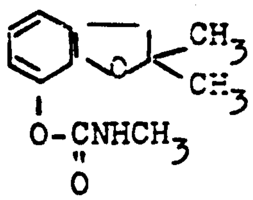 (Carbofuran)

C. 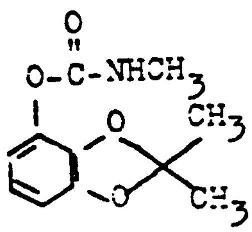 (Bendiocarb)

D. 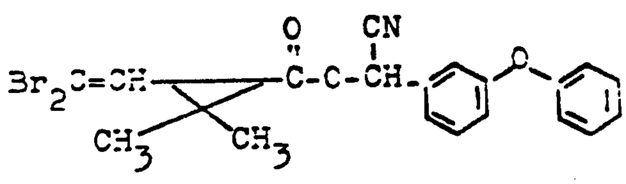 (Decamethrin)

E. 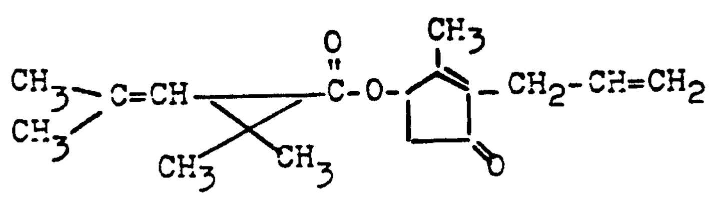 (d-trans-Allethrin)

F. O

$C\ Cl_2=CH-O-P-(OCH_3)_2$ (Dichlorvos)

Tabelle 2

Beispiele für erfindungsgemäß verwendbare Synergisten

$$R^1-\overset{R}{\underset{R^2}{C}}-(CH_2)_m-NH-COOCH_2-C\equiv CH$$

| Synergist Nr. | R | $R^1$ | $R^2$ | n | Verbindung aus Herstellungsbeispiel Nr. |
|---|---|---|---|---|---|
| 1 | $CH_2F$ | $CH_2F$ | $CH_2F$ | 0 | 3 |
| 2 | $CH_2Cl$ | $CH_2Cl$ | $CH_2Cl$ | 0 | 4 |
| 3 | $CH_2F$ | $CH_2F$ | $CH_2F$ | 1 | 1 |
| 4 | $CH_2F$ | $CH_3$ | $CH_2F$ | 1 | 5 |
| 5 | $CH_2F$ | $CH_3$ | $CH_3$ | 1 | 6 |
| 6 | $CH_2Cl$ | $CH_3$ | $CH_2Cl$ | 0 | 7 |
| 7 | $CH_3$ | $CH_2Cl$ | $CH_3$ | 0 | 8 |
| 8 | $CH_2F$ | $CH_2F$ | $CH_3$ | 0 | 2 |
| 9 | $CH_2F$ | $CH_3$ | $CH_3$ | 0 | 9 |
| 10 | Piperonylbutoxid (Stand der Technik) | | | | |

Tabelle 3

Testergebnisse

| Wirkstoff | | Synergist | Konzentration in % Wirkstoff | Synergist | LT 100 in Minuten oder bei 360' in % |
|---|---|---|---|---|---|
| A | | - | 1,0 | - | 360' = 60 % |
| B | | - | 0,2 | - | 360' = 50 % |
| C | | - | 1,0 | - | 360' = 90 % |
| D | | - | 0,0016 | - | 150' |
| E | | - | 0,04 | - | 120' |
| F | | - | 0,008 | - | 105' |
| - | | 1 | - | 0,2 | 360' = 90 % |
| - | | 2 | - | 1,0 | 360' = 40 % |
| - | | 3 | - | 1,0 | 360' = 35 % |
| - | | 4 | - | 0,2 | 360' = 90 % |
| - | | 5 | - | 0,2 | 360' |
| - | | 6 | - | 0,2 | 180' |
| - | | 7 | - | 0,2 | 210' |
| - | | 8 | - | 0,2 | 360' = 20 % |
| - | | 9 | - | 0,2 | 360' = 20 % |
| - | | 10 | - | 1,0 | 360' = 0 % |
| A | + | 1 | 0,04 + | 0,04 | 90' |
| A | + | 2 | 0,04 + | 0,04 | 105' |
| A | + | 3 | 0,04 + | 0,04 | 90' |
| A | + | 4 | 0,04 + | 0,04 | 90' |
| A | + | 5 | 0,04 + | 0,04 | 75' |
| A | + | 6 | 0,04 + | 0,04 | 75' |
| A | + | 7 | 0,04 + | 0,04 | 90' |
| A | + | 8 | 0,04 + | 0,04 | 90' |
| A | + | 9 | 0,04 + | 0,04 | 75' |
| A | + | 10 | 0,04 + | 0,04 | 360' = 80 % |

| Wirkstoff | | Synergist | Konzentration in % Wirkstoff | Synergist | LT 100 in Minuten oder bei 360' in % |
|---|---|---|---|---|---|
| B | + | 1 | 0,04 + | 0,04 | 90' |
| B | + | 2 | 0,04 + | 0,04 | 90' |
| B | + | 3 | 0,04 + | 0,04 | 90' |
| B | + | 4 | 0,04 + | 0,04 | 75' |
| B | + | 5 | 0,04 + | 0,04 | 90' |
| B | + | 6 | 0,04 + | 0,04 | 75' |
| B | + | 7 | 0,04 + | 0,04 | 75' |
| B | + | 8 | 0,04 + | 0,04 | 90' |
| B | + | 9 | 0,04 + | 0,04 | 90' |
| B | + | 10 | 0,04 + | 0,04 | 360' = 45 % |
| C | + | 1 | 0,04 + | 0,04 | 120' |
| C | + | 2 | 0,04 + | 0,04 | 150' |
| C | + | 3 | 0,04 + | 0,04 | 150' |
| C | + | 4 | 0,04 + | 0,04 | 120' |
| C | + | 5 | 0,04 + | 0,04 | 90' |
| C | + | 6 | 0,04 + | 0,04 | 90' |
| C | + | 7 | 0,04 + | 0,04 | 120' |
| C | + | 8 | 0,04 + | 0,04 | 120' |
| C | + | 9 | 0,04 + | 0,04 | 120' |
| C | + | 10 | 0,04 + | 0,2 | 360' = 90 % |
| D | + | 2 | 0,0016 | + 0,0016 | 105' |
| D | + | 4 | 0,0016 | + 0,0016 | 90' |
| D | + | 5 | 0,0016 | + 0,0016 | 105' |
| D | + | 6 | 0,0016 | + 0,0016 | 120' |
| D | + | 7 | 0,0016 | + 0,0016 | 105' |
| D | + | 8 | 0,0016 | + 0,0016 | 105' |
| D | + | 9 | 0,0016 | + 0,0016 | 105' |
| D | + | 10 | 0,0016 | + 0,0016 | 150' |

| Wirkstoff | | Synergist | Konzentration in % Wirkstoff | Synergist | LT 100 in Minuten oder bei 360' in % |
|---|---|---|---|---|---|
| E | + | 2 | 0,04 + | 0,04 | 45' |
| E | + | 4 | 0,04 + | 0,04 | 45' |
| E | + | 5 | 0,04 + | 0,04 | 30' |
| E | + | 6 | 0,04 + | 0,04 | 45' |
| E | + | 7 | 0,04 + | 0,04 | 45' |
| E | + | 8 | 0,04 + | 0,04 | 45' |
| E | + | 10 | 0,04 + | 0,04 | 60' |
| F | + | 3 | 0,008 + | 0,008 | 75' |
| F | + | 4 | 0,008 + | 0,008 | 75' |
| F | + | 6 | 0,008 + | 0,008 | 75' |
| F | + | 8 | 0,008 + | 0,008 | 75' |
| F | + | 9 | 0,008 + | 0,008 | 75' |
| F | + | 10 | 0,008 + | 0,008 | 90' |

Die Herstellung der erfindungsgemäßen Verbindungen soll anhand der folgenden Herstellungsbeispiele erläutert werden.

Beispiel 1

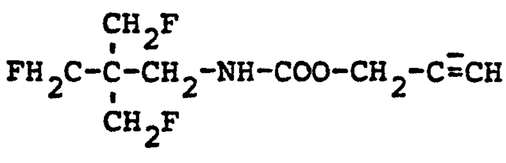

N-(2,2-Bisfluormethyl-3-fluorpropyl)-carbamidsäure-O-2-propinylester

16,7 g 2,2-Bisfluormethyl-3-fluorpropyl-isocyanat (29 %ige Lösung in Toluol) werden in 100 ml Toluol gelöst und 10 mg Diazabicyclooctan (Dabco) zugesetzt. Zu diesem Reaktionsgemisch tropft man bei Raumtemperatur (ca. 20°C) 5,6 g 2-Propinyl-alkohol. Nach vierstündigem Erhitzen des Gemisches zum Sieden wird das Reaktionsgemisch abgekühlt und mit Wasser gewaschen. Nach Trocknen über Natriumsulfat wird das Lösungsmittel abdestilliert und das zurückbleibende Öl im Hochvakuum vom restlichen Lösungsmittel befreit. Man erhält 22 g N-(2,2-Bisfluormethyl-3-fluorpropyl)-carbamidsäure-O-2-propinylester in Form eines farblosen Öls (80 % der Theorie Ausbeute) $n_D^{20}$: 1,4505

Beispiel 2

N-(1,1-Bisfluormethyl-ethyl)-carbamidsäure-O-2-propinylester

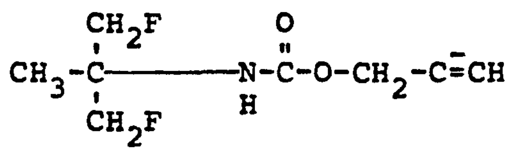

14 g (0,1 mol) 2,2-Bisfluormethylpropansäurefluorid werden in 200 ml Aceton gelöst. Hierzu tropft man bei 0°C eine Lösung von 6,5 g (0,1 mol) Natriumazid in 20 ml Wasser, rührt eine Stunde bei Raumtemperatur und gibt 200 ml Wasser zu. Die wäßrige Phase wird zweimal mit je 200 ml Toluol extrahiert und die resultierende vereinte Toluolphase zweimal mit je 200 ml Wasser gewaschen. Nach Trocknen über Natriumsulfat wird die Toluolphase langsam bis zum Ende der auftretenden Gasentwicklung auf 70-80°C erhitzt. Anschließend kocht man eine Stunde am Rückfluß. Die Vollständigkeit der Bildung des 1,1-Bisfluormethyl-ethylisocyanats wird IR-spektroskopisch verfolgt (Abnahme $\tilde{\nu}_{CON_3} = 2120\ cm^{-1}$, Zunahme $\tilde{\nu}_{N=C=O} = 2250\ cm^{-1}$). Zu der auf diese Weise erhaltene Lösung des 1,1-Bisfluormethyl-ethylisocyanates tropft man 11,2 g 2-Propinylalkohol, gibt 10 mg Diazabicyclooctan (Dabco) zu und verfährt weiter wie unter Herstellungsbeispiel 1.

Man erhält 13 g N-(1,1-Bisfluormethyl-ethyl)-carbamidsäure-O-2-propinylester in Form eines farblosen Öls (68 % der Theorieausbeute) $n_D^{20}$: 1,4430

Die Verbindungen der folgenden Beispiele werden analog den Beispielen 1 und 2 erhalten:

$$R^1-\overset{\displaystyle R}{\underset{\displaystyle R^2}{C}}-(CH_2)_n-NH-COOCH_2-C\equiv CH$$

| Beispiel Nr. | R | $R^1$ | $R^2$ | n | Brechungsindex $n_D^{20}$ bzw. Fp.°C |
|---|---|---|---|---|---|
| 3 | $CH_2F$ | $CH_2F$ | $CH_2F$ | 0 | 1,4371 |
| 4 | $CH_2Cl$ | $CH_2Cl$ | $CH_2Cl$ | 0 | 55 |
| 5 | $CH_2F$ | $CH_3$ | $CH_2F$ | 1 | 1,4505 |
| 6 | $CH_3$ | $CH_2F$ | $CH_3$ | 1 | 1,4580 |
| 7 | $CH_2Cl$ | $CH_3$ | $CH_2Cl$ | 0 | 1,4952 |
| 8 | $CH_3$ | $CH_2Cl$ | $CH_3$ | 0 | 1,4757 |
| 9 | $CH_2F$ | $CH_3$ | $CH_3$ | 0 | 1,4470 |

Die Herstellung der erfindungsgemäß verwendbaren Zwischenprodukte (bzw. deren Vorprodukte) kann gemäß den folgenden Beispielen erfolgen:

Beispiel 1A

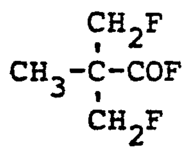 aus 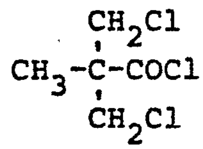

Es werden 947,5 g (5 mol) Dichlorpivaloylchlorid, 2 kg Tetramethylensulfon und 1,16 kg (20 mol) Kaliumfluorid bei einem Anfangsdruck von 2 bar $N_2$ für 5 Stunden bei 230°C in einem VA-Rührautoklaven erhitzt, abgekühlt, entspannt und unter leicht vermindertem Druck destilliert. Man erhält 576 g (79,2 % der Theorie) Difluorpivaloylfluorid ($Kp._{110-120\ mbar}$ 52-56°C). Im 3 kg Maßstab in einem Schaufelreaktor erhält man Ausbeuten zwischen 85 und 90 % der Theorie.

Beispiel 2A

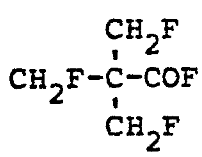 aus 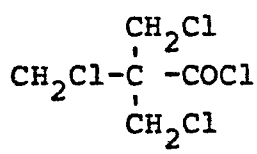

Es werden 900 g (4 mol) Trischlormethylessigsäurechlorid (Trichlorpivaloylchlorid), 1,16 kg (20 mol) Kaliumfluorid und 2250 ml Tetramethylensulfon unter Normaldruck 5 Stunden bei 200°C gerührt, abgekühlt, mit 1 l Xylol versetzt und bis zum Siedepunkt des Tetramethylensulfons abdestilliert. Die Xylollösung enthält das gesamte Trisfluormethylessigsäurefluorid (80 % der Theorie), das wegen seines Schmelzpunktes von 50-52°C des Siedepunkts von 48°C/22 mbar und der hohen Reaktivität des Säurefluorids im allgemeinen in der Lösung direkt weiter umgesetzt wird.

Beispiel 3A

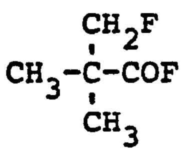

aus

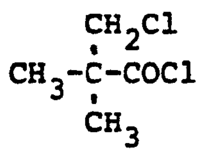

5,8 kg (100 mol) Kaliumfluorid und 7,7 kg Tetramethylensulfon werden in einem 20 l-Schaufelreaktor mit Destillationsaufbau vorgelegt und bei ca. 20 mbar unter Abnahme von 10 % des eingesetzten Lösungsmittels andestilliert. Es wird mit $N_2$ belüftet, die Innentemperatur von 150° auf 125-130°C fallengelassen, 6,2 kg (40 mol) Chlorpivaloylchlorid eingesaugt, die Apparatur mit $N_2$ gespült und druckdicht verschlossen. Nach Aufpressen von 3 bar $N_2$ wird 1 Stunde auf 150°C und 12 Stunden auf 230°C erwärmt, auf 80°C abgekühlt und bei 100 mbar destilliert. Man erhält

3,256 kg (68 % der Theorie) Fluorpivaloylfluorid (Siedepunkt 40-41°C/100 mbar) und 1,4 kg (26 %) Chlorpivaloylfluorid (Siedepunkt 65°C/100 mbar) als Nebenprodukt. Das Ergebnis entspricht einer Selektivität von 92 % bei 74 % Umsatz.

Beispiel 1B

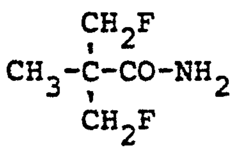
aus
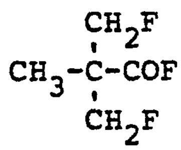

Es werden 700 g 25 %ige (Gew.-%) wäßrige Ammoniak-Lösung und 500 g Eis vorgelegt und 515 g (3,7 mol) Difluorpivalsäurefluorid (Bisfluormethylpropionsäurefluorid) gelöst in 250 ml $CH_2Cl_2$ zugetropft. Man läßt bei ca. 10°C 15 Minuten nachreagieren, saugt den Niederschlag ab, wäscht mit etwas kaltem Wasser und trocknet bei 70°C unter vermindertem Druck. Ausbeute: 451 g (89 % der Theorie) Bisfluormethylpropionsäureamid;
Fp.: 100-101°C

Beispiel 2B

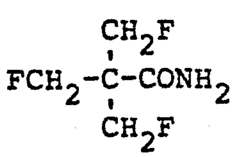
aus
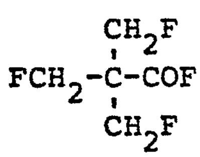

In analoger Weise wie im Beispiel 1 beschrieben, jedoch mit Xylol als Lösungsmittel wird bei einer Reaktionstemperatur von 40-50°C aus Trifluormethylessigsäurefluorid (Trifluorpivalsäurefluorid) in einer Ausbeute > 95 % der Theorie Trifluormethylessigsäureamid erhalten.
Fp.: 114-115°C.

<u>Beispiel 3B</u>

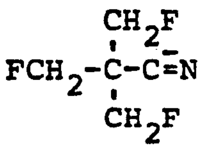 aus 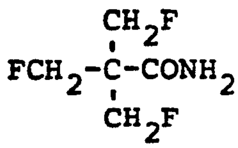

Es werden 250 g (1,6 mol) Trifluormethyl-essigsäureamid und 284 g (2 mol) $P_2O_5$ gut durchmischt und bei ca. 70 mbar und 160-200°C Außentemperatur erhitzt, wobei das Nitril abdestilliert. Der Versuch wird in dem gleichen Reaktionsgefäß mit dem Rückstand wiederholt und man erhält insgesamt 434 g (98 % der Theorie) Trisfluormethylacetonitril; Kp.: 90°C/58 mbar; Fp.: 70-75°C.

<u>Beispiel 4B</u>

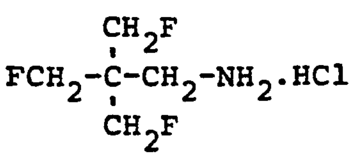 aus 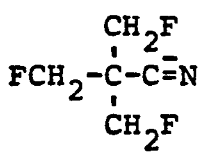

30 g (0,218 mol) Trisfluormethylessigsäurenitril werden in 180 ml Methanol gelöst und in Gegenwart von 10 g Raney-Nickel (Ni:Fe 85:15) bei 80-90 bar $H_2$ und einer Temperatur von 38-40°C hydriert. Es wird filtriert und das Filtrat im Wasserstrahlvakuum bis zur Trockne von Methanol befreit. Man erhält 36 g (93 % der Theorie) Trifluorneopentylaminhydrochlorid.

Beispiel 5B

$$\begin{array}{ccc} \quad CH_2F & & \quad CH_2F \\ FCH_2-C-CH_2-NH_2 & \text{aus} & FCH_2-C-CH_2NH_2 \cdot HCl \\ \quad CH_2F & & \quad CH_2F \end{array}$$

106,5 g (0,6 mol) Trifluorneopentylaminhydrochlorid werden unter Eiskühlung in eine Lösung von 200 g 50 %igem KOH in 40 ml $H_2O$ eingetragen, das freie Amin mit 250 ml Ether ausgeschüttelt, mit $MgSO_4$ getrocknet, eingeengt und der feste Rückstand unter vermindertem Druck 1 Stunde bei 20°C getrocknet. Ausbeute an freiem Amin: 66 g (78 % der Theorie); Fp.: 66-67°C; Kp.: 74°C/52 mbar.

Beispiel 6B

$$\begin{array}{ccc} \quad CH_2F & & \quad CH_2F \\ CH_3-C-CH_2-NCO & \text{aus} & CH_3-C-CH_2-NH_2 \cdot HCl \\ \quad CH_3 & & \quad CH_3 \end{array}$$

Es werden 300 ml Chlorbenzol bei 20°C mit Phosgen ($COCl_2$) gesättigt, 110,6 g (0,7 mol) 3-Fluor-2,2-dimethylpropyl-aminhydrochlorid (Monofluorneopentylaminhydrochlorid) zugegeben und unter stetiger Phosgenzufuhr langsam bis zum Rückfluß erhitzt. Nach Beendigung der HCl-Abspaltung wird noch ca. 2 Stunden $N_2$ durchgeleitet und dann destilliert.
Ausbeute an Monofluorneopentylisocyanat: 95 g (92 % der Theorie); Kp.: 52°C/20 mbar.

Die übrigen erfindungsgemäß verwendbaren Zwischenprodukte (bzw. deren Vorprodukte) können analog erhalten werden.

<u>Patentansprüche</u>

1. Halogenalkylcarbamidsäureester der allgemeinen Formel (I)

$$R^1-\overset{\displaystyle R}{\underset{\displaystyle R^2}{\overset{|}{\underset{|}{C}}}}-(CH_2)_n-NH-CO-O-\overset{\displaystyle R^3}{\underset{\displaystyle R^4}{\overset{|}{\underset{|}{C}}}}-C\equiv C-R^5 \qquad (I)$$

in welcher

$R$, $R^1$ und $R^2$ unabhängig voneinander für gegebenenfalls durch Halogen substituiertes Methyl stehen, wobei wenigstens eine der Methylgruppen $R$, $R^1$ und $R^2$ durch wenigstens ein Halogenatom substituiert ist und

$R^3$, $R^4$ und $R^5$ gleich oder verschieden sind und einzeln für Wasserstoff oder für gegebenenfalls substituiertes Alkyl stehen, und

n für 0 oder 1 steht.

2. Halogenalkylcarbamidsäureester der allgemeinen Formel (I) gemäß Anspruch 1, in welcher

R,$R^1$ und $R^2$ unabhängig voneinander für gegebenenfalls durch Fluor oder Chlor substituiertes Methyl stehen, wobei wenigstens eine der Methylgruppen R, $R^1$ und $R^2$ durch wenigstens ein Fluor- oder Chloratom substituiert ist, und

$R^3$,$R^4$ und $R^5$ gleich oder verschieden sind und einzeln für Wasserstoff oder für einen gegebenenfalls durch Halogen, Cyano, $C_1$-$C_4$-Alkoxy, $C_1$-$C_4$-Alkylthio oder Trifluormethyl substituierter Alkylrest mit 1-2 Kohlenstoffatomen stehen, und

n für 0 oder 1 steht.

3. Halogenalkylcarbamidsäureester der allgemeinen Formel (I) gemäß Anspruch 1, in welcher

R,$R^1$ und $R^2$ die in Anspruch 1 oder 2 angegebene Bedeutung haben, und

$R^3$,$R^4$ und $R^5$ für Wasserstoff stehen, und

n für 0 oder 1 steht.

4. Halogenalkylcarbamidsäureester der allgemeinen Formel (I) gemäß Anspruch 1, in welcher

| | |
|---|---|
| R, $R^1$ und $R^2$ | unabhängig voneinander für $CH_3$, $CH_2Cl$ und/oder $CH_2F$ stehen, wobei wenigstens einer der Reste R, $R^1$ und $R^2$ für $CH_2Cl$ oder $CH_2F$ steht, und |
| $R^3$, $R^4$ und $R^5$ | für Wasserstoff stehen, und |
| n | für 0 oder 1 steht. |

5. Halogenalkylcarbamidsäureester der allgemeinen Formel (I) gemäß Anspruch 1, in welcher

| | |
|---|---|
| R, $R^1$ und $R^2$ | unabhängig voneinander für $CH_3$ oder $CH_2F$ stehen, wobei wenigstens einer der Reste R, $R^1$ und $R^2$ für $CH_2F$ steht, und |
| $R^3$, $R^4$ und $R^5$ | für Wasserstoff stehen, und |
| n | für 0 oder 1 steht. |

6. Verfahren zur Herstellung von Halogenalkylcarbamidsäureestern der allgemeinen Formel (I)

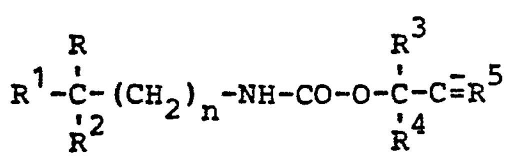

(I)

in welcher

$R, R^1$ und $R^2$ unabhängig voneinander für gegebenenfalls durch Halogen substituiertes Methyl stehen, wobei wenigstens eine der Methylgruppen R, $R^1$ und $R^2$ durch wenigstens ein Halogenatom substituiert ist, und

$R^3, R^4, R^5$ gleich oder verschieden sind und einzeln für Wasserstoff oder für gegebenenfalls substituiertes Alkyl stehen, und

n für 0 oder 1 steht,

dadurch gekennzeichnet, daß man Alkylisocyanate der allgemeinen Formel (II)

$$R^1-\overset{R}{\underset{R^2}{C}}-(CH_2)_n-NCO \qquad (II)$$

in welcher

$R, R^1, R^2$ und n die oben angegebene Bedeutung haben,

mit Alkinylalkoholen der allgemeinen Formel (III)

$$HO-\overset{R^3}{\underset{R^4}{C}}-C\equiv C-R^5 \qquad (III)$$

in welcher

$R^3$,$R^4$ und $R^5$ die oben angegebene Bedeutung haben,

gegebenenfalls in Gegenwart eines aprotischen Verdünnungsmittels und gegebenenfalls in Gegenwart eines Katalysators bei Temperaturen zwischen 0 und 120°C umsetzt.

7. Schädlingsbekämpfungsmittel, gekennzeichnet durch einen Gehalt an wenigstens einem Halogenalkylcarbamidsäureester der allgemeinen Formel (I) gemäß Anspruch 1 oder 6.

8. Verwendung der Halogenalkylcarbamidsäureester der allgemeinen Formel (I) gemäß den Ansprüchen 1 oder 6 für die Schädlingsbekämpfung.

9. Verfahren zur Herstellung von Schädlingsbekämpfungsmitteln, dadurch gekennzeichnet, daß man wenigstens einen arthropodiziden Wirkstoff und wenigstens einen Synergisten der allgemeinen Formel (I) (gemäß Anspruch 1 oder 6) mit den üblichen Streck- und Verdünnungsmitteln sowie gegebenenfalls Formulierhilfsstoffen, wie Emulgatoren, vermischt.

10. Verfahren zur Bekämpfung von Schädlingen, dadurch gekennzeichnet, daß man Schädlingsbekämpfungsmittel gemäß Anspruch 7 auf die Schädlinge und/oder ihren Lebensraum einwirken läßt.